# EUROPEAN PATENT SPECIFICATION

(11) **EP 1 603 565 B1**
(45) Date of publication and mention of the grant of the patent: **23.07.2008**
(21) Application number: 04715295.4
(22) Date of filing: 27.02.2004
(51) Int. Cl.: A61K 31/47, A61P 11/08

(54) **MEDICAMENT COMPRISING A HIGHLY POTENT LONG-LASTING BETA2-AGONIST IN COMBINATION WITH OTHER ACTIVE INGREDIENTS**
MEDIKAMENT ENTHALTEND EIN HOCHAKTIVES LANGWIRKENDES BETA-2-AGONIST IN VERBINDUNG MIT ANDEREN WIRKSTOFFEN
MEDICAMENT COMPORTANT UN AGONISTE BETA 2 DE LONGUE DUREE ET TRES PUISSANT EN COMBINAISON AVEC D'AUTRES INGREDIENTS ACTIFS

(30) Priority: 27.02.2003 EP 03004184
(43) Date of publication of application: 14.12.2005
(60) Divisional application: 07009204.4
(73) Proprietor: CHIESI FARMACEUTICI S.p.A., I-43100 Parma (IT)
(72) Inventor: RAZZETTI, Roberta, I-43100 Parma (IT); PASTORE, Fiorella, I-43100 Parma (IT)
(74) Representative: Bianchetti, Giuseppe
(86) International application number: PCT/EP2004/001960
(87) International publication number: WO 2004/075896

(56) References cited:
- EP-A- 1 157 689
- WO-A-01/78693
- WO-A-98/41193
- US-A- 5 874 063
- US-A- 6 030 604
- [Online] XP002282993 Retrieved from the Internet: URL:www.chiesegroup.com/news/news-detail-e ng.asp?id=41> [retrieved on 2002-10-06]
- EUROPEAN RESPIRATORY JOURNAL SUPPL 50 vol. 28, 2006, pages 666 - 666
- DONNEL ET AL: 'Bronchodilator activity of single doses of a new long acting beta2 agonists TA-2005 in male asthmatics' EUROPEAN RESPIRATORY JOURNAL SUPPL 19 vol. 8, 1995, pages P1300 - P1300

## Description

### BACKGROUND OF THE INVENTION

Asthma is a disease which is becoming more prevalent and is the most common disease of childhood. It can be identified by recurrent wheeze and intermittent air flow limitation. Despite many advances in its understanding, said pathology remains a poorly understood and often poorly treated disease. Previously, contraction of airway smooth muscles has been regarded as the most important feature of asthma. Recently there has been a marked change in the way asthma is managed, stemming from the fact that asthma is recognized as a chronic inflammatory disease. Uncontrolled airway inflammation may lead to mucosal damage and structural changes giving irreversible narrowing of the airways and fibrosis of the lung tissue. Therapy should therefore be aimed at controlling symptoms so that normal life is possible and at the same time provide basis for treating the underlying inflammation.

The symptoms may be controlled by first generation β₂-adrenoceptor agonists such as salbutamol, fenoterol and terbutalin or second generation ones such as formoterol and salmeterol (long-acting β₂-agonists) which overcome the disadvantage of the short duration of action particularly for patients with nocturnal asthma. Prophylactic therapy, instead, is typically provided by corticosteroids such as beclometasone dipropionate, fluticasone propionate mometasone furoate and budesonide.

Another respiratory disease whose incidence is steadily increasing throughout the world is chronic obstructive pulmonary disease (COPD). Most patients with COPD have acquired their lung disease through smoking cigarettes. Depending upon trends in tobacco smoking, it is set to rise to fifth most prevalent cause of disability, worldwide by 2020 (Leckie M et al Exp Opin Invest Drugs 2000, 9, 3-23).

Chronic obstructive pulmonary disease (COPD) is defined as a disease state **characterized by** the presence of airflow obstruction due to chronic bronchitis or emphysema.

Chronic bronchitis is **characterized by** excessive secretion of bronchial mucus, whereas emphysema denotes abnormal, permanent enlargement of air spaces distal to the terminal bronchiole, with destruction of their walls and without obvious fibrosis (American Thoracic Society). Each condition is treated as specific diseases.

Chronic obstructive bronchiolitis is due to obstruction of the peripheral airways as a result of inflammation in the bronchioles.

Drugs intended for the treatment of lung diseases such as asthma and COPD are currently administered by pulmonary delivery which relies on inhalation of an aerosol through the mouth and throat so that the drug substance can reach the lung. They can be administered as aqueous or hydroalcoholic formulations through a nebuliser, as dry powders by means of Dry Powder Inhalers or in halogenated hydrocarbon propellants. The propellant-based systems require suitable pressurized metered-dose inhalers (pMDIs) which release a metered dose of medicine upon each actuation.

Complicated therapy with different medications and devices may lead to poor compliance of the patients, so to under-treatment and, in turn, negative impact on their quality of life. This is dramatically evident in the case of long-term management of chronic asthma, in particular with prophylactic treatments, such as inhaled steroids, which do not give immediate symptom relief. Recent therapeutic strategy is aimed at both controlling the symptoms and reducing the inflammation by fixed combinations of a long-acting β₂-agonist and a corticosteroid.

Combinations containing salmeterol and fluticasone propionate, both under the form of dry powder and HFA formulations, are currently on the market under the trade name of Seretide^{®}. Each dose of the combined formulations is administered twice-daily. Each inhalation from the powder formulation can deliver a nominal dose of 50 microg of *rac*-salmeterol xinafoate and one of three doses of fluticasone propionate 100, 250 and 500 microg. Using the HFA formulation, each puff from the inhaler can deliver a nominal dose of 25 microg of salmeterol and 50, 125 or 250 microg of fluticasone.

A combination containing formoterol and budesonide in form of dry powder is currently on the market under the trade name of Symbicort^{®} and each single dose is administered twice-daily. Each inhalation can deliver a nominal dose of 6 microg of *rac*-formoterol fumarate and either 100 or 200 microg of budesonide.

Several articles in the scientific literature deal with the use of β₂-agonists in combination with other classes of drugs, in particular corticosteroids and anticholinergics.

Moreover, various kinds of combinations have been proposed in the patent literature.

Nevertheless, it has never been demonstrated in the prior art that, by using a long-acting β₂-agonist (LABA) in the combination therapy, an increment both of the bronchodilating and of the anti-inflammatory efficacy can be achieved so as making possible to reduce the dose without affecting the therapeutic effect.

In this respect, for example, it would be highly advantageous to provide a combination of a β₂-agonist and a steroid which: i) while keeping the rapid onset of action, has a longer duration of action in such a way as that the formulation can be administered once a day so delaying the possible appearance of tolerance towards the β₂-agonist and with a great improvement of the compliance of patients, in particular of those with chronic and nocturnal asthma; *ii)* allows to reduce the dose of corticosteroid.

2(1H)-quinolone derivatives have been disclosed in the past, for instance in EP 147719 and WO 00/75114, and characterized as potent long lasting bronchodilating agents useful for the treatment or prophylaxis of various chronic obstructive pulmonary disease.

A medicament comprising a particular 2(1H)-quinolinone derivative LABA and a corticosteroid for the treatment of inflammatory or obstructive airways diseases has been recently disclosed in WO 02/45703. In the description it is stated in general terms that it is possible to use said combination to reduce the dosages of corticosteroid required for a given therapeutic effect compared with those required using treatment with a corticosteroid alone, but no supporting evidence is reported.

8-Hydroxy-5-[(1R)-1-hydroxy-2-[[(1R)-2-(4-methoxyphenyl)-1-methylethyl] amino]ethyl-2(1H)-quinolinone hydrochloride salt, known with the experimental code TA 2005 is a highly potent long-acting β₂-agonist also **characterized by** a rapid onset of action, disclosed for the first time in EP 147719. In the description it is stated that the compound can be administered either orally or parenterally. As far as the daily dose is concerned, only a very broad generic range is reported, i.e. 0.01 to 30 µg, especially 0.01 to 3 µg per kg of body weight (corresponding to about 0.7 to 2100 µg, especially 0.7 to 210 µg). JP 09-309830 referred to its use as an anti-inflammatory agent by inhalation but even in this case, a broad generic range of doses was reported, i.e. from 1 to 20 µg, for example about 3 to 10 µg.

In Eur Resp J 8 (Suppl 19), 1995, 258s, the results of two clinical studies were shown. In the latter (P1300), mild asthmatics inhaled single doses of 6 and 9 µg TA 2005 and placebo from a metered dose nebuliser system at one week intervals. At these two dose levels TA 2005 produced a rapid and long lasting bronchodilation. The former (P1301) concerned a randomised, double bind, placebo-controlled, rising dose, safety study. Single doses of either 0.8, 1.6, 3.2, 6.4, 9.6, 12.8 µg were inhaled by healthy male volunteers from a metered dose nebuliser system. TA 2005 caused a dose-dependent increase in heart rate, tremor and pulmonary function and a decrease in plasma potassium. According to the authors, the maximum no adverse effect dose of TA 2005 was 9.6 µg. A very large therapeutic window between 0.8 and 9.6 µg has been therefore suggested, but an efficacious and safe dose has not been identified.

In EP 1157689 the applicant described aerosol pharmaceutical compositions comprising a β₂-agonist belonging to the class of phenylalkylamino derivatives in solution in a HFA propellant and a co-solvent whose apparent pH was adjusted to between 2.5 and 5.0 in order to guarantee an adequate shelf-life of the medicament. In the description, it has been stated that TA 2005 formulations will be advantageously suitable for delivering 2-10 µg/dose, preferably 3-5 µg/dose. A 3.5 µg/dose HFA 134a formulation containing 12% w/w ethanol and 1.0% IPM has been reported in example 7.

Several other patents or patent applications, i.e. US 6221398, US 5874063, US 6030604, WO 98/41193, WO 98/31352 , WO 01/78693, WO 01/89480 and WO 03/080939 mention TA 2005 in a list of possible β₂-agonists in compositions comprising other classes of drugs such as corticosteroids, anticholinergics or phosphodiesterase-4-inhibitors.

In none of the documents of the prior art, specific combination products of TA 2005 with other active ingredients have been disclosed provided with such a beneficial pharmacological profile as the combination products of the invention.

### OBJECT OF THE INVENTION

The present invention provides a medicament comprising, separately or together: 8-hydroxy-5-[(1R)-1-hydroxy-2-[[(1R)-2-(4-methoxyphenyl)-1-methyl ethyl]amino]ethyl-2(1H)-quinolinone and/or a physiologically acceptable salt and/or solvate thereof (compound A), 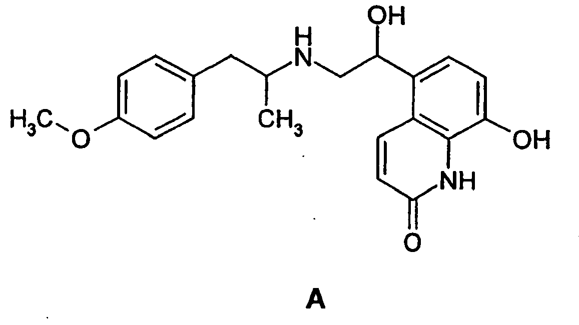 and budesonide and its epimers, for simultaneous, sequential or separate administration in the prophylaxis or treatment of an inflammatory or obstructive airways disease such as asthma or COPD.

In a further aspect, the present invention provides a pharmaceutical composition comprising, together, effective amounts of (A) as hereinbefore defined and budesonide or its epimers, as hereinbefore defined, optionally together with at least one pharmaceutically acceptable carrier.

The invention further provides the use of (A) as hereinbefore defined in combination with budesonide or its epimers, as hereinbefore defined in the preparation of a pharmaceutical composition or a kit for the prophylaxis or treatment, by simultaneous, sequential or separate administration of (A) and the other active substances, of any inflammatory or obstructive airways disease.

It has indeed been found that, upon combination of such β₂-agonist and budesonide both the bronchodilator and the anti-inflammatory effects increase.

The current pharmacological therapies for COPD are aimed at relieving symptoms and reducing exacerbations. Bronchodilators cause only a little improvement in spirometric measurements, but may improve symptoms and exercise tolerance and reduce the infective exacerbations.

In COPD patients, the contribute of anticholinergic/antimuscarinic drugs is important, because they relieve the airways constriction due to the vagal cholinergic tone. In fact, as demonstrated in the examples that follow, TA 2005 gives rise to synergistic interaction in controlling plasma exudations in the airways.

Accordingly, it might be possible to reduce in the combination products of the invention, for any active ingredient, the dose recommended for each component without affecting the therapeutic effect, so diminishing the risk of appearance of the side-effects associated to its use.

In a particular aspect, the present invention provides a medicament wherein the compound (A) is present in the combination as hydrochloride salt (TA 2005) in a suitable amount to provide a daily dose comprised between 1 and 4 µg, for simultaneous, sequential or separate administration once or twice daily, preferably once daily, in the treatment of an inflammatory or obstructive airways disease such as asthma or COPD.

Effective dose of TA 2005 in the combination product is 1 µg.

It has indeed been found that at these dosage strength TA 2005 is therapeutically effective upon inhalation.

The bronchodilator efficacy of TA 2005, defined as increase from baseline in the forced expiratory volume over one second (FEV1), measured by spirometry, has been assessed in a preliminary clinical trial in a small group of asthmatic patients.

The results have shown that already following administration of a single dose of 1 µg a mean and persistent increase of FEV1 approximately 30% greater than baseline can be obtained.

As said before, compound (A) is preferably used in the form of its hydrochloride salt (TA 2005). Other suitable physiological salts of compound (A) include bromide, sulphate, phosphate, maleate, fumarate, tartrate, citrate, benzoate, mesilate, acorbate, salicylate, acetate, succinate, lactate, glutarate or gluconate. Solvates of salts such as hydrates, emihydrates or others with pharmaceutically acceptable organic solvents are also comprised in the invention.

The medicament of the invention can comprise anticholinergic atropine-like derivatives such as ipratropium bromide, oxitropium bromide, tiotropium bromide or glycopyrrolate or its 3R,2'R-enantiomer, in order to provide a medicament particularly effective for the treatment of COPD.

The preferred anticholinergic, antimuscarinic agent is tiotropium bromide.

The pharmaceutical combinations according to the invention may contain compound (A) and budesonide in ratio by weights ranging from 1:1600 to 1:50, preferably from 1:1000 to 1:50.

The intended dose regimen is twice or once daily, preferably once daily, where the suitable daily dose of compound (A) is advantageously in the range of 1 to 4 µg, depending on the patient (age, weight and so on) and on the kind and the severity of the disease.

The combination of the invention is preferably administered by inhalation. Inhalable preparations include inhalable powders, propellant-containing metering aerosols or propellant-free inhalable formulations.

For administration as a dry powder, single- or multi-dose inhalers known from the prior art may be utilized, wherein the powder can be filled in gelatine, plastic or other capsules, cartridges or blister packs or in a reservoir.

A diluent or carrier, generally non-toxic and chemically inert to the medicaments, e.g. lactose or any other additive suitable for improving the respirable fraction can be added to the powdered medicament.

Inhalation aerosols containing propellant gas such as hydrofluoroalkanes may contain the active ingredients of the combination of the invention either in solution or in dispersed form.

The propellant-driven formulations may also contain other ingredients such as co-solvents, stabilizers and optionally other excipients.

The propellant-free inhalable formulations comprising the combination of the invention may be in form of solutions or suspensions in an aqueous, alcoholic or hydroalcoholic medium and they may be delivered by jet or ultrasonic nebulizers known from the prior art or by soft-mist nebulizers such as Respimat^{®}.

Treatment of inflammatory or obstructive airways diseases in accordance with the invention may be symptomatic or prophylactic. Inflammatory or obstructive airways diseases to which the claimed combinations are applicable include asthma of whatever type or genesis including both intrinsic (non-allergic) asthma and extrinsic (allergic) asthma, mild asthma, moderate asthma, severe asthma, bronchitic asthma, exercise-induced asthma, occupational asthma and asthma induced following bacterial infection. Treatment of asthma is also to be understood as embracing treatment of subjects, e.g. of less than 4 or 5 years of age, exhibiting wheezing symptoms and diagnosed or diagnosable as "wheezy infants", an established patient category of major medical concern. Prophylactic efficacy in the treatment of asthma will be evidenced by reduced frequency or severity of symptomatic attack, e.g. of acute asthmatic or bronchoconstrictor attack, improvement in lung function or improved airways hyperreactivity. It may further be evidenced by reduced requirement for other, symptomatic therapy. Prophylactic benefit in asthma may in particular be apparent in subjects prone to "morning dipping". "Morning dipping" is a recognized asthmatic syndrome, common to a substantial percentage of asthmatics and **characterized by** asthma attack, e.g. between the hours of about 4 to 6 a.m., i.e. at a time normally substantially distant from any previously administered symptomatic asthma therapy.

Other inflammatory or obstructive airways diseases and conditions to which the present invention is applicable include acute lung injury (ALI), adult respiratory distress syndrome (ARDS), chronic obstructive pulmonary, airways or lung disease (COPD, COAD or COLD) including chronic bronchitis and emphysema, bronchiolitis, bronchiectasis and exacerbation of airways hyperreactivity consequent to other drug therapy, in particular other inhaled drug therapy.

Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tobacosis and byssinosis.

The invention further provides a pharmaceutical kit comprising compound (A) and budesonide or epimers thereof, as hereinbefore described, in separate unit dosage forms, said forms being suitable for administration of (A) and the combination compound in effective amounts. Such a kit suitably further comprises one or more inhalation devices for administration of (A) and the combination compound. For example, the kit may comprise one or more dry powder inhalation devices adapted to deliver dry powder from a capsule, together with capsules containing a dry powder comprising a dosage unit of (A) and capsules containing a dry powder comprising a dosage unit of the combination compound. In another example, the kit may comprise a multidose dry powder inhalation device containing in the reservoir thereof a dry powder comprising (A) and a multidose dry powder inhalation device containing in the reservoir thereof a dry powder comprising the combination compound. In a further example, the kit may comprise a metered dose inhaler containing an aerosol comprising comprising (A) in a propellant and a metered dose inhaler containing an aerosol comprising the budesonide in a propellant.

The invention is illustrated with reference to the examples which follow.

The unexpected features of the combination products of TA 2005 with budesonide according to the present invention is demonstrated in an in vivo model of Sephadex-induced lung inflammation according to the method reported by Kublin R et al Int Arch Allergy Immunol 1992; 98: 266-272; in an in vitro assay on human U-937 derived macrophage cell line as described in Naunyn-Schmiedeberg's Arch. Pharmacol., 362:184-189, 2000; as well as in a model of acetaldehyde induced bronchoconstriction (Berti et al., Arzneim-Forsch/Drug Res 1994; 44: 323-326).

### Example 1 - In vitro assay on human U-937 derived macrophage cell line of the anti-inflammatory efficacy of the combination of the invention

U-937 cells were cultured and macrophage-differentiated by 10 ng/ml phorbol myristate acetate for 48 h.

The cells were then incubated with 1 µg/ml lipopolysaccharide (LPS) or LPS together with concentration ranges of β₂-agonist, corticosteroid, or combination. After different times of incubation TNFα and IL-10 were measured. Then the medium was collected and the cells lysed for cyclic adenosine 3',5'-monophosphate (cAMP) measurement.

TNFα and IL-10 in the culture medium were determined by a commercial available ELISA assay, while cAMP was determined by a commercial ³H-cAMP test system.

Dose-response curve of corticosteroid was determined in the absence and in the presence of TA 2005 given at the concentrations of 10⁻¹⁰ and 10⁻⁸ M. Budesonide alone inhibited both TNFα and IL-10 release without affecting cAMP content. The addition of TA 2005 potentiated the effect on TNFα and counteracted the negative effect of budesonide on IL-10 release.

Interestingly the addition of TA 2005 resulted in a strong stimulation of cAMP by budesonide.

### Example 2 - In vivo assay of the anti-inflammatory efficacy of the combination of the invention in a Sephadex- induced lung oedema model

The rat lung oedema induced by Sephadex is a model which leads to inflammatory cells infiltration and long-lasting interstitial oedema. The antiinflammatory activity of TA 2005 alone and in combination with budesonide in comparison with another long-lasting β₂-adrenoceptor agonist, formoterol, was evaluated.

Anaesthetized rats (200-250 g) were dosed intratracheally with Sephadex beads (5 mg/ml) at a dose volume of 1 ml/kg. Control group received 1 ml/kg saline.

The test substances were suspended in saline and administered intratracheally suitably diluted in the Sephadex suspension.

24 h post-administration, the animals were sacrificed and the lungs removed and weighted. Percent inhibition of the Sephadex-induced oedema was then determined.

Intratracheal instillation of Sephadex beads induced a statistically significant increase in lung weight compared to control animals (33 ± 3%). Treatment with TA 2005 (0.001, 0.01, 0.05 and 0.1 nmol/kg), formoterol (0.01, 0.1, 1 and 2 nmol/kg) and budesonide (15, 30, 60, 120, 240 and 480 nmol/kg) produced a dose-dependent remission of Sephadex-induced lung oedema.On the basis of the dose-response curves, non effective doses of TA 2005 (0.01 nmol/kg) and formoterol (0.1 nmol/kg) were chosen to be combined with low, non effective doses of budesonide (15, 30, 60 and 120 nmol/kg).In the presence of TA 2005, budesonide was able to significantly inhibit the Sephadex-induced lung oedema already at a dose as low as 15 nmol/kg, while in its absence 240 nmol/kg was needed. In the presence of formoterol the potentiation was much less evident, as a significant reduction in the Sephadex-induced lung weight increase was observed only with the 120 nmol/kg dose of corticosteroid.

### Example 3 - Effects of TA 2005 and budesonide combination on the bronchoconstriction induced by acetaldehyde in the guinea pig

The ability of TA 2005 to control the bronchoconstriction and neurogenic inflammation elicited by acetaldehyde (AcCHO) has been investigated in anaesthetized artificially ventilated guinea pigs, following the experimental model described by Berti et al., Arzneim-Forsch/Drug Res 1994; 44: 323-326.

Intravenous injection of AcCHO induced a dose-dependent bronchoconstriction, accompanied by increase in blood histamine and Evans blue extravasation in the tracheal tissue, indicating alteration of vascular permeability.

The protective activity of TA 2005 (0.1 to 10 pmol), formoterol (0.3 to 30 pmol) or budesonide (31.25 to 500 nmol) was determined after intratracheal superfusion alone or in combination.

All the effects of AcCHO were potently antagonised by TA 2005 and formoterol. However, TA 2005 was almost two fold as potent as formoterol in preventing bronchoconstriction, release of histamine and plasma extravasation. TA 2005 displayed also a significantly longer duration of action at all doses selected. For example, when the two β₂-adrenoceptor agonists were compared at almost equieffective doses as a peak (10 and 30 pmol), the anti-bronchoconstrictive effect of TA--2005 was still fully present after 240 min, whereas the effect of formoterol declined approximately by 50%.

The corticosteroid budesonide was also capable to lessen the effect of AcCHO in the guinea pig airways, but as expected, its efficacy was in the nanomolar range and noteworthy inferior to that observed with both TA 2005 and formoterol.

The tracheal superfusion of TA 2005 in combination with budesonide gave rise to a synergistic interaction in dominating the three main effects of AcCHO such as bronchoconstriction, histamine release and plasma extravasation in guinea pig airway. Considering the parameter bronchoconstriction, the ED₅₀ value of budesonide alone (396 nmol) was reduced 5.3 and 14.3 fold when it was combined with 0.1 and 0.3 pmol of TA 2005, respectively. A synergistic interaction was achieved also by adding formoterol to the corticosteroid. However, this combination was less effective than that obtained with TA 2005. In fact the ED₅₀ value of budesonide was reduced 3.0 and 5.9 fold when it was combined with 0.3 and 1 pmol of formoterol, respectively.

### Example 4 - Effects of budesonide on the TA 2005-induced desensitisation in isolated tracheal strips from ovalbumin-sensitised guinea pigs

A prolonged use of β₂-agonists results in down-regulation of pulmonary β₂-adenoceptors. This phenomenon can be counteracted by concomitant treatment with a corticosteroid.

In the present study tracheal strips obtained from ovalbumin-sensitised guinea-pigs (100 mg/kg ip and 100 mg/kg sc, 20 days before sacrifice) were submitted to β₂-desensitisation by contact for two 20-min periods with a supra-maximal concentration of salbutamol (5*10⁻⁶ M). In some experiments guinea pigs 24 and 1.5 h before sacrifice received 50 mg/kg ip of budesonide.

After β₂-desensitisation, TA 2005 resulted about 3 times less potent in relaxing the carbachol-induced contraction. Budesonide pretreatment reversed the rightward shift of the TA 2005 dose-response curves and even potentiated by about 6 times its relaxing effects.

### Example 7 - Formulation for metered dose inhaler comprising TA 2005 and Budesonide

A HFA formulation for metered dose inhaler was prepared with the composition as follows:

| *Components* | *Amounts* | | |
|---|---|---|---|
| | Per unit | | Nominal dose |
| | mg | % | µg |
| TA 2005 | 0.15 | 0.0016 w/v | 1 |
| Budesonide | 30.00 | 0.317 w/v | 200 |
| Ethanol | 1650.0 | 15 w/w | - |
| pH adjusted to about 3.5 - 4.0 | | | - |
| Water (optional) | 220.05 | 2.0 w/w | |
| HFA 134a q.s. to 9.45 ml | 9114.5 | - | - |

The pH of the formulation has been adjusted with a suitable amount of a mineral acid.

The formulation (120 actuations/canister, overage of 30 actuations) was filled in aluminum canisters having the internal surface coated with Teflon (two stage pressure filling) and fitted with a metering valve having a 63 µl metering chamber.

### Example 8 - Powder formulation for a dry powder inhaler comprising TA 2005 and Budesonide

A powder formulation for dry powder inhaler was prepared with the composition as follows:

| *Components* | *Amounts* | |
|---|---|---|
| | Per unit dose | |
| | mg | % |
| TA 2005 | 0.001 | |
| Budesonide | 0.400 | |
| Alpha-lactose monohydrate 212-355 µm | 8.6151 | 86.2 |
| Pre-blend | 0.9839 | 10 |
| | | |
| Total weight | 10 | |

### Preparation of the formulation

The pre-blend mixture was obtained as follows: alpha-lactose monohydrate SpheroLac 100 (Meggle EP D30) with a starting particle size of 50 to 400 µm and magnesium stearate with a starting particle size of 3 to 35 µm in the ratio 98:2 w/w were co-milled in a jet mill apparatus.

About 86% w/w alpha-lactose monohydrate CapsuLac (212 - 355 µm) was placed in a 2.5 l stainless steel container, then about 10% w/w of the pre-blend mixture was added. The powder was mixed in a Turbula mixer for 4 h at 32 r.p.m. TA 2005 and budesonide were added to the powder and mixed in a Turbula mixer to obtain, respectively, a ratio of 1 µg and 400 µg of the active ingredients to 10 mg of carrier.

## Claims

1. A pharmaceutical composition comprising, combined together, an effective amount of 8-Hydroxy-5-((1R)-1-hydroxy-2-[[(1R)-2-(4-methoxyphenyl)-1-methylethyl]amino]ethyl-2(1H)-quinolinone (compound A) and budesonide or an epimer thereof for simultaneous use in the treatment of an inflammatory or obstructive airways disease, wherein the effective amount of the compound (A) is 1 µg per unit dose and the ratio by weight between compound A and budesonide or epimer thereof is from 1:1000 to 1:50.

2. A composition according to claim 1 wherein the compound (A) is in the form of hydrochloride salt.

3. A composition according to claim 1 or 2 in which the budesonide epimer is the 22-R epimer.

4. A composition according to any of the claims from 1 to 3 further comprising an antimuscarinic agent

5. A pharmaceutical composition according to any of the claims from 1 to 4, wherein said composition is an inhalatory composition.

6. A pharmaceutical composition according to claim 5 to be administered by pressurized metered dose inhalers wherein the active ingredients are in solution or suspension in a propellant gas.

7. A pharmaceutical composition according to claim 5 to be administered by a nebulizer comprising a solution or a suspension of the compound (A) and budesonide or epimer thereof in an aqueous, alcoholic or hydroalcoholic medium.

8. A pharmaceutical composition according to claim 5 to be administered by a dry powder inhaler in which the compound (A) and budesonide or epimer thereof are in dry powder form.

## Patentansprüche

1. Pharmazeutische Zusammensetzung, umfassend, kombiniert miteinander, eine wirksame Menge an 8-Hydroxy-5-[(1R)-1-hydroxy-2-[[(1R)-2-(4-methoxyphenyl)-1-methylethyl]amino]-ethyl]-2(1H)-chinolinon (Verbindung A) und Budesonid oder ein Epimer davon zur gleichzeitigen Verwendung in der Behandlung einer inflammatorischen oder obstruktiven Atemwegserkrankung, wobei die wirksame Menge der Verbindung (A) 1 µg pro Einzeldosis ist und das Gewichtsverhältnis zwischen Verbindung A und Budesonid oder Epimer davon 1:1.000 bis 1:50 ist.

2. Zusammensetzung nach Anspruch 1, wobei die Verbindung (A) in der Hydrochloridsalz-Form ist.

3. Zusammensetzung nach Anspruch 1 oder 2, wobei das Budesonid-Epimer das 22-R-Epimer ist.

4. Zusammensetzung nach einem der Ansprüche 1 bis 3, die außerdem ein Antimuscarinikum umfasst.

5. Pharmazeutische Zusammensetzung nach einem der Ansprüche 1 bis 4, wobei die Zusammensetzung eine Zusammensetzung zum Inhalieren ist.

6. Pharmazeutische Zusammensetzung nach Anspruch 5, die mit Treibgasdosieraerosolen ("pressurized metered dose inhalers") zu verabreichen ist, wobei die aktiven Ingredientien in Lösung oder Suspension in einem Treibgas sind.

7. Pharmazeutische Zusammensetzung nach Anspruch 5, die durch einen Vernebler zu verabreichen ist, die eine Lösung oder eine Suspension der Verbindung (A) und Budesonid oder Epimer davon in einem wässrigen, alkoholischen oder wässrigalkoholischen Medium umfasst.

8. Pharmazeutische Zusammensetzung nach Anspruch 5, die durch einen Trockenpulverinhalator zu verabreichen ist, in der die Verbindung (A) und Budesonid oder Epimer davon in Trockenpulverform sind.

## Revendications

1. Composition pharmaceutique comprenant, en combinaison, une quantité efficace de 8-hydroxy-5-[(1R)-1-hydroxy-2-[(1R)-2-(4-méthoxyphényl)-1-méthyléthyl]amino]éthyl-2-(1H)-quinolinone (composé A) et du budénoside ou un épimère de celui-ci pour utilisation simultanée dans le traitement d'une maladie inflammatoire ou obstructive des voies aériennes, dans laquelle la quantité efficace du composé (A) est de 1 µg par dose unitaire et le rapport pondéral entre le composé A et le budénoside ou un épimère de celui-ci est de 1 : 1000 à 1 : 50.

2. Composition selon la revendication 1 dans laquelle le composé (A) est sous la forme d'un sel de chlorhydrate.

3. Composition selon la revendication 1 ou 2 dans laquelle l'épimère du budénoside est l'épimère 22-R.

4. Composition selon l'une quelconque des revendications 1 à 3 comprenant en outre un agent antimuscarinique.

5. Composition pharmaceutique selon l'une quelconque des revendications 1 à 4, ladite composition étant une composition inhalatoire.

6. Composition pharmaceutique selon la revendication 5 à administrer par des aérosols-doseurs pressurisés dans lesquels les principes actifs sont en solution ou en suspension dans un gaz propulseur.

7. Composition pharmaceutique selon la revendication 5 à administrer par un nébuliseur comprenant une solution ou une suspension du composé (A) et de budénoside ou d'un épimère de celui-ci dans un milieu aqueux, alcoolique ou hydroalcoolique.

8. Composition pharmaceutique selon la revendication 5 à administrer par un inhalateur de poudre sèche dans lequel le composé (A) et le budénoside ou un épimère de celui-ci sont sous forme de poudre sèche.
